# EUROPEAN PATENT SPECIFICATION

(11) **EP 1 351 972 B1**
(45) Date of publication and mention of the grant of the patent: **01.03.2006**
(21) Application number: 01270543.0
(22) Date of filing: 05.12.2001
(51) Int. Cl.: C07J 41/00, A61K 31/15, C12N 15/63

(54) **LIPIDS COMPRISING AN AMINOXY GROUP**
LIPIDE MIT AMINOXYGRUPPE
LIPIDES COMPRENANT UN GROUPE AMINOXY

(30) Priority: 12.12.2000 WO PCT/GB00/04767; 06.06.2001 GB 0113781
(43) Date of publication of application: 15.10.2003
(73) Proprietor: MITSUBISHI CHEMICAL CORPORATION, Tokyo 108-0014 (JP)
(72) Inventor: JORGENSEN, Michael, IC Vec Limited, Armstrong Road, South Kensington, London (GB); KELLER, Michael, IC Vec Limited, Armstrong Road, South Kensington, London (GB); MILLER, Andrew David, IC Vec Limited, Armstrong Road, South Kensington, London (GB); PEROUZEL, Eric, IC Vec Limited, Armstrong Road, South Kensington, London (GB)
(74) Representative: Alcock, David
(86) International application number: PCT/GB2001/005385
(87) International publication number: WO 2002/048170

(56) References cited:
- WO-A-97/45442
- WO-A-98/28317
- GB-A- 1 178 790
- GB-A- 1 288 647
- US-A- 3 766 235
- US-A- 4 189 431
- US-A- 5 283 185
- US-A- 5 510 510
- LEE E R ET AL: "DETAILED ANALYSIS OF STRUCTURES AND FORMULATIONS OF CATIONIC LIPIDS FOR EFFICIENT GENE TRANSFER TO THE LUNG" HUMAN GENE THERAPY, XX, XX, vol. 7, no. 14, 10 September 1996 (1996-09-10), pages 1701-1717, XP002035784 ISSN: 1043-0342
- GROCHOWSKI E ET AL: "A convenient sysnthesis of steroidoxyamines" POLISH JOURNAL OF CHEMISTRY, POLISH CHEMICAL SOCIETY, XX, vol. 52, no. 2, 1978, pages 335-339, XP002194243
- DATABASE CROSSFIRE BEILSTEIN [Online] BEILSTEIN INSTITUT ZUR FOERDERUNG DER WISSENSCHAFTEN, FRANKFURT, DE; BRNs: 6086579, 1882795,1876909,1876140,1817271, XP002194245 & MAMALIS P ET AL: JOURNAL OF THE CHEMICAL SOCIETY, 1960, pages 229-238,
- DATABASE BIOSIS [Online] BIOSCIENCES INFORMATION SERVICE, PHILADELPHIA, PA, US; 1979 MOFFETT R B ET AL: "ANTI ULCER AGENTS 2. PYRIDYL OXIME ETHERS" Database accession no. PREV198070004469 XP002194246 & JOURNAL OF HETEROCYCLIC CHEMISTRY, vol. 16, no. 7, 1979, pages 1459-1468, ISSN: 0022-152X -& DATABASE CROSSFIRE BEILSTEIN [Online] BEILSTEIN INSTITUT ZUR FOERDERUNG DER WISSENSCHAFTEN, FRANKFURT, DE; BRN: 1477032, XP002194247
- CRICCHIO R ET AL: "OXIMES OF 3-FORMYLRIFAMYCIN SV.SYNTHESIS, ANTIBACTERIAL ACTIVITY, AND OTHER BIOLOGICAL PROPERTIES" JOURNAL OF MEDICINAL CHEMISTRY, AMERICAN CHEMICAL SOCIETY. WASHINGTON, US, vol. 17, 1974, pages 396-403, XP001000166 ISSN: 0022-2623
- ROSE K ET AL: "NATURAL PEPTIDES AS BUILDING BLOCKS FOR THE SYNTHESIS OF LARGE PROTEIN-LIKE MOLECULES WITH HYDRAZONE AND OXIME LINKAGES" BIOCONJUGATE CHEMISTRY, AMERICAN CHEMICAL SOCIETY, WASHINGTON, US, vol. 7, 1996, pages 552-556, XP001015560 ISSN: 1043-1802
- CHRISTIE W W: "What is a Lipid? Definitions" [Online] XP002194244 Retrieved from the Internet: <URL: http://www.lipid.co.uk/infores/Lipids/what lip/file.pdf> [retrieved on 2002-03-14] page 1 -page 11

## Description

The present invention relates to a compound.

One aspect of gene therapy involves the introduction of foreign nucleic acid (such as DNA) into cells, so that its expressed protein may carry out a desired therapeutic function.

Examples of this type of therapy include the insertion of TK, TSG or ILG genes to treat cancer, the insertion of the CFTR gene to treat cystic fibrosis; the insertion of NGF, TH or LDL genes to treat neurodegenerative and cardiovascular disorders; the insertion of the IL-1 antagonist gene to treat rheumatoid arthritis; the insertion of HIV antigens and the TK gene to treat AIDS and CMV infections; the insertion of antigens and cytokines to act as vaccines; and the insertion of β-globin to treat haemoglobinopathic conditions, such as thalassaemias.

Many current gene therapy studies utilise adenoviral gene vectors - such as Ad3 or Ad5 - or other gene vectors. However, serious problems have been associated with their use. This has prompted the development of less hazardous, non-viral approaches to gene transfer.

A non-viral transfer system of great potential involves the use of cationic liposomes. In this regard, cationic liposomes - which usually consist of a neutral phospholipid and a cationic lipid - have been used to transfer DNA, mRNA, antisense oligonucleotides, proteins, and drugs into cells. A number of cationic liposomes are commercially available and many new cationic lipids have recently been synthesised. The efficacy of these liposomes has been illustrated by both *in vitro* and *in vivo.*

A cytofectin useful in the preparation of a cationic liposome is *N*-[1-(2,3-dioleoyloxy)propyl]-*N,N,N*-trimethyl ammonium chloride, otherwise known as "DOTMA".

One of the most commonly used cationic liposome systems consists of a mixture of a neutral phospholipid dioleoylphosphatidylethanolamine (commonly known as "DOPE") and a cationic lipid, 3β-[(*N*,*N*-dimethylaminoethane)carbamoyl]cholesterol (commonly known as "DC-Chol").

Despite the efficacy of the known cationic liposomes there is still a need to optimise the gene transfer efficiency of cationic liposomes in human gene therapy. With the near completion of the human genome project, the use of genes for therapeutic purposes, described as gene therapy is increasingly expected to revolutionise medicine. In this context, even though still less effective than viral technology, non-viral delivery is increasingly recognised by the scientific community as the safest option for human applications.

This field has evolved considerably in the last decade with the apparition of complex macromolecular constructs including many elements of different existing technologies (viral proteins or peptides, liposomes, polymers, targeting strategies and stealth properties).

Our copending application PCT/GB00/04767 teaches a system based on a triplex composed of a viral core peptide Mu, plasmid DNA and cationic Liposome (LMD). This platform technology gave us good success *in vitro* and promising results *in vivo.* But as for all existing non-viral technology more development is needed to achieve a therapeutic level *in vivo.*

To this end, formulation must achieve stability of the particle in biological fluids (serum, lung mucus) and still maintain efficient transfection abilities.

This requirement is one of the main hurdles of all existing technology. Current stable formulations achieve little transfection and most present efficient transfecting agents are drastically limited in the scope of their application due to this instability.

After administration (in blood for systemic application or in mucus for lung topical administration), the charged complexes are exposed to salt and biological macromolecules leading to strong colloidal aggregation and adsorption of biological active elements (opsonins) at their surface. The gene vehicles undergo drastic changes that could include precipitation, binding of proteins leading to particle elimination by macrophages and surface perturbation resulting in its destruction.

With the aim of generating drug and gene delivery systems for cell specific targeting *in vitro* and *in vivo,* protocols are required for the production of biological fluid-stable delivery systems with sufficient activity to exhibit therapeutic benefits. Therefore, a balance between stability and activity must be found for an efficient drug/gene delivery vehicle.

Our copending applications PCT/GB00/04767 teaches a system based on modified lipid wherein the lipid carries a carbohydrate moiety. These modified lipids have been found to stable and have low toxicity. Such systems require the linking an additional moiety to the lipid to assist in the provision of a modified lipid which is stable and has low toxicity. There is a desire in the art to provide lipids comprising groups to which additional moieties may be readily linked. Also, modified lipids comprising a lipid linked to an amino headgroup via a linker or a spacer arm have been disclosed in Human Gene Therapy 7:1701-1717 (1996), US-A-5283185 and WO-A-97/45442.

WO-A-98/28317 has suggested the use of oxime linkers to attach a wide range of groups to each other, and the use of oxime linkers with lipids have been disclosed in J. Medicinal Chemistry, 17: 396-403 (1974) and J. Heterocycl. Chem. 16: 1459-1463 (1979).

The present invention alleviates the problems of the prior art.

According to one aspect of the present invention there is provided a process for preparing a compound of the formula 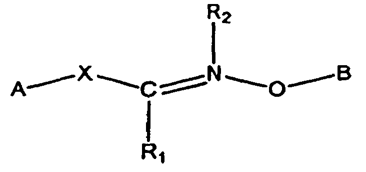 comprising reacting (i) a compound of the formula; and 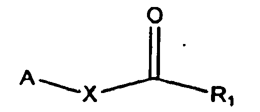 (ii) a compound of the formula 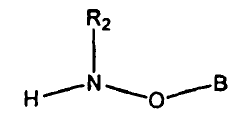
in admixture with or associated with a nucleotide sequence, or a pharmaceutically active agent; wherein B is a lipid and A is a moiety of interest (MOI); wherein × is an optional linker group; wherein R₁ is H or a hydrocarbyl group; and wherein R₂ is a lone pair or R₄, wherein R₄ is a suitable substituent.

According to one aspect of the present invention there is provided a composition comprising (i) a compound of the formula 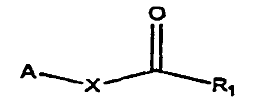
(ii) a compound of the formula 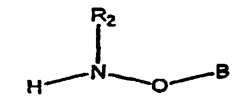
and (iii) a nucleotide sequence, or a pharmaceutically active agent;
wherein B is a lipid and A is a moiety of interest (MOI); wherein X is an optional linker group; wherein R₁ is H or a hydrocarbyl group; and wherein R₂ is a lone pair or a suitable substituent.

A composition of the present invention may be used in therapy.

A composition of the present invention may be used in the manufacture of a medicament for the treatment of a genetic disorder or a condition or a disease.

A liposome may be formed from a composition of the present invention.

A method of preparing such a liposome comprises forming the liposome from a composition of the present invention.

Such a liposome may be used in therapy.

Such a liposome may be used in the manufacture of a medicament for the treatment of genetic disorder or condition or disease.

A pharmaceutical composition may be formed comprising a composition of the present invention that is admixed with a pharmaceutical and, optionally, admixed with a pharmaceutically acceptable diluent, carrier or excipient

A pharmaceutical compositon may be formed comprising a liposome as described above and admixed with a pharmaceutical and, optionally, admixed with a pharmaceutically acceptable diluent, carrier or excipient.

Some further aspects of the invention are defined in the appended claims.

We have found the provision of a lipid comprising an aminoxy group allows for simple linking of further moieties to the lipid *via* the aminoxy group. When reacted with a moiety (MOI) comprising an aldehyde or ketone group, a compound is provided in which the MOI and lipid are linked via an amide group. Such a linkage may be simple prepared in a "one-pot" reaction. This methodology avoids extensive purification procedures by simple dialysis or excess, non-reacted reagents.

The post-coating one-pot methodology of the present process is based on selective and high reactivity of the aminoxy-linker to react with aldehydes and ketones to form -C=N-(Schiff-base like) covalent linkages. Importantly, the reaction can be carried out in aqueous environment at basic or acidic pH. Furthermore, there is no partial breakdown of the reactive group when exposed to aqueous conditions as it is the case for NHS-activated carboxyls and other esters. Therefore, the stability of the reactive species, e.g. the aldehyde/ketone and the aminoxy allows total control of the surface reaction without loss of reactive species due to hydrolysis/degradation.

### PREFERRED ASPECTS

The compound of the present invention is of the formula 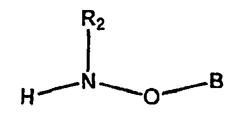
wherein B is a lipid; and wherein R₂ is H or a hydrocarbyl group.

The term "hydrocarbyl group" as used herein means a group comprising at least C and H and may optionally comprise one or more other suitable substituents. Examples of such substituents may include halo, alkoxy, nitro, an alkyl group, a cyclic group etc. in addition to the possibility of the substituents being a cyclic group, a combination of substituents may form a cyclic group. If the hydrocarbyl group comprises more than one C then those carbons need not necessarily be linked to each other. For example, at least two of the carbons may be linked via a suitable element or group. Thus, the hydrocarbyl group may contain hetero atoms. Suitable hetero atoms will be apparent to those skilled in the art and include, for instance, sulphur, nitrogen and oxygen. A nonlimiting example of a hydrocarbyl group is an acyl group.

A typical hydrocarbyl group is a hydrocarbon group. Here the term "hydrocarbon" means any one of an alkyl group, an alkenyl group, an alkynyl group, which groups may be linear, branched or cyclic, or an aryl group. The term hydrocarbon also includes those groups but wherein they have been optionally substituted. If the hydrocarbon is a branched structure having substituent(s) thereon, then the substitution may be on either the hydrocarbon backbone or on the branch; alternatively the substitutions may be on the hydrocarbon backbone and on the branch.

Preferably the reaction of the present invention is performed in an aqueous medium.

### OPTIONAL LINKER X

In a preferred aspect optional linker X is present.

In a preferred aspect X is a hydrocarbyl group.

in a preferred aspect the linker X comprises or is linked to the lipid via a polyamine group.

It is believed that the polyamine group is advantageous because it increases the DNA binding ability and efficiency of gene transfer of the resultant liposome.

In one embodiment, preferably the polyamine group is a unnaturally occurring polyamine. It is believed that the polyamine head-group is advantageous because the increased amino functionality increases the overall positive charge of the liposome. In addition, polyamines are known to both strongly bind and stabilise DNA. In addition, polyamines occur naturally in cells and so it is believed that toxicological problems are minimised.

In another embodiment, preferably two or more of the amine groups of the polyamine group of the present invention are separated by one or more groups which are not found in nature that separate amine groups of naturally occurring polyamine compounds (i.e. preferably the polyamine group of the present invention has un-natural spacing).

Preferably the polyamine group contains at least two amines of the polyamine group that are separated (spaced from each other) from each other by an ethylene (-CH₂CH₂-) group.

Preferably each of the amines of the polyamine group are separated (spaced from each other) by an ethylene (-CH₂CH₂-) group.

Typical examples of suitable polyamines include spermidine, spermine, caldopentamine, norspermidine and norspermine. Preferably the polyamine is spermidine or spermine as these polyamines are known to interact with single or double stranded DNA. An alternative preferred polyamine is caldopentamine.

### R₁

In a preferred aspect R₁ is H

### C=N

The C=N bond may be acid labile or acid resistant.

In one aspect the C=N bond is acid labile.

In one aspect the C=N bond is acid resistant.

### MOI

The moiety of interest (MOI) may be any moiety which one wishes to link to a lipid.

The MOI may be a carbohydrate moiety.

In a preferred aspect the carbohydrate moiety is a mono-saccharide.

In a preferred aspect the carbohydrate moiety is a sugar moiety.

Preferably the carbohydrate moiety is selected from mannose, glucose (D-glucose), galactose, glucuronic acid, lactose, maltose, maltotriose, maltotetraose, maltoheptaose and mixtures thereof. More preferably the carbohydrate moiety is D-glucose.

In one aspect the compound of the present invention comprises from 1 to 7 carbohydrate moieties. Preferably the compound comprises one carbohydrate moiety.

### LIPID

In a preferred aspect the lipid is or comprises a cholesterol group or a glycerol/ceramide backbone. Any lipid-like structure or polyamine is suitable.

Preferably the cholesterol group is cholesterol.

Preferably the cholesterol group is linked to X *via* a carbamoyl linkage.

The cholesterol group can be cholesterol or a derivative thereof. Examples of cholesterol derivatives include substituted derivatives wherein one or more of the cyclic CH₂ or CH groups and/or one or more of the straight-chain CH₂ or CH groups is/are appropriately substituted. Alternatively, or in addition, one or more of the cyclic groups and/or one or more of the straight-chain groups may be unsaturated.

In a preferred embodiment the cholesterol group is cholesterol. It is believed that cholesterol is advantageous as it stabilises the resultant liposomal bilayer.

Preferably the cholesterol group is linked to the optional linker group *via* a carbamoyl linkage. It is believed that this linkage is advantageous as the resultant liposome has a low or minimal cytotoxicity.

### Further Aspects

Preferably R₂ is H or a hydrocarbyl group.

In a preferred aspect the R₂ hydrocarbyl group contains optional heteroatoms selected from O, N and halogens.

In a preferred aspect R₂ is H.

Preferably the process of the present invention is an aqueous medium or in a wholly aqueous medium.

The present invention further provide a compound prepared by a process of the present invention defined herein, a compound obtained by a process of the present invention defined herein, and/or a compound obtainable by a process of the present invention defined herein.

Preferably the compound is in admixture with or associated with a nucleotide sequence.

The nucleotide sequence may be part or all of an expression system that may be useful in therapy, such as gene therapy.

In a preferred aspect the compound of the present invention is in admixture with a condensed polypeptide/ nucleic acid complex to provide a non-viral nucleic acid delivery vector. The condensed polypeptide/ nucleic acid complex preferably include those disclosed in our copending application PCT/GB00/04767. Preferably the polypeptides or derivatives thereof are capable of binding to the nucleic acid complex. Preferably the polypeptides or derivatives thereof are capable of condensing the nucleic acid complex. Preferably the nucleic acid complex is heterologous to the polypeptides or derivatives thereof.

Preferably the process comprises the use of a molecular sieve.

Preferably, the cationic liposome is formed from the compound of the present invention and a neutral phospholipid - such as DOTMA or DOPE. Preferably, the neutral phospholipid is DOPE.

The present invention will now be described in further detail by way of example only with reference to the accompanying figures in which:-
**Figure 1** - Scheme 1 Synthesis of Hydroxylamine lipid **11**. *Reagents:* (a) CH₂Cl₂, Et₃N, Boc₂O, rt, 5h, 98%; (b) EtOAc, N-hydroxysuccinimide (1 eq.), DCC (1 eq.), 10 h., rt; (c) **(8)**, EtOAC/THF [95/5], Et₃N (pH = 8), 2 h., r.t, 90%; (d) CH₂Cl₂, TFA (15 eq), 0°C, N₂, 5 h, 86%.
**Figure 2 -** Principle of chemioselective glycosylation of O-substituted hydroxylamine with D-Glucose (Although the β-anomer is shown, mutarotation does occur and α-anomer is produced as well).
**Figure 3 -** Possible structures of neoglycolipid obtained from mannose.
**Figure 4 -** Result of analysis of differents lipoplexes size by photon correlation spectroscopy (PCS). The size was measured after 30 min for lipoplexes at [DNA]=1 µg/ml in Optimem +/- 10% FCS, 37°C. The comparison of standard LMD formulation (LMD) and LMD modified by addition of 7.5 molar % of product **12h** and **12i** was made in Optimem (white) and 10% Serum (black) and expressed in percent of size increase over the original measured size of 180 nm .
**Figure 5 -** A comparison between the transfection efficiencies of basic LMD and LMD glycomodified with 7.5 molar % of product **12h** and **12i** onto Hela Cells in 0%(white). 50%(black and white) and 100 % Serum (black) conditions. The results are expressed as relative light units per milligram of protein (n = 4).

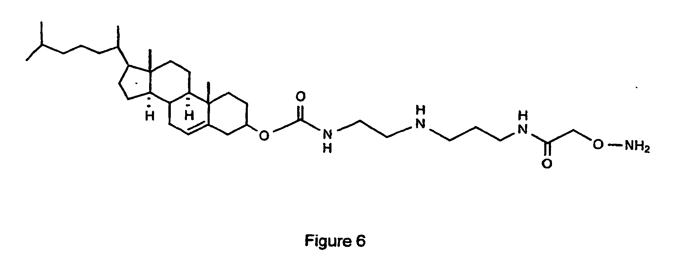

**Figure 6 -** A structure of an aminoxy lipid

The following examples describe the synthesis of compounds (i) and (ii) in the absence of a nucleotide sequence or a pharmaceutically active agent as required by the present claims.

### EXAMPLES

### Experimental Section

### Synthesis of neoglycolipids

**General:**^{**1**}**H** NMR spectra were recorded at ambient temperature on either Brucker DRX400, DRX300 or Jeol GX-270Q spectrometers, with residual nonisotopicaly labeled solvent (e.g. CHCl3, δ_{H}=7.26) as an internal reference. ¹³C-NMR spectra were recorded on the same range of spectrometers at 100, 75 and 68.5 MHz respectively, also with residual nonisotopicaly labelled solvent (e.g. CHCl₂, δ_{C}=77.2) as an internal reference. Infrared Spectra were recorded on Jasco FT/IR 620 using NaCl plates and Mass spectra (Positive ions electrospray) were recorded using VG-7070B or JEOL SX-102 instruments. Chromatography refers to flash column chromatography, which was performed throughout on Merck-Kieselgel 60 (230-400 mesh) with convenient solvent. Thin layer chromatography (Tlc) was performed on pre-coated Merck-Kieselgel 60 F254 aluminium backed plated and revealed with ultraviolet light, iodine, acidic ammonium molybdate(IV), acidic ethanolic vanilin, or other agents as appropriate. Neoglycolipids purity was assessed using analytical high-pressure liquid chromatography (HPLC) on a Hitachi system using a Purospher® RP-18 endcapped column (5 µm). Elution was performed at an isocratic flow rate of 1 mL/min with CH₃CN/H₂O (60:40) and fraction were detected at 205 nm wavelength before collection and Mass Analysis. Dried CH2Cl2 was distilled with phosphorous pentoxide before use. All other dry solvents and chemicals were purchased from Sigma-Aldrich Company LTD (Poole, Dorset, UK).

**Abbreviations:** Boc: tert-butoxycarbonyl ; br: broad ; Chol: cholesteryl ; DMF: *N,N-*dimethyl formamide ; DMSO: dimethyl sulfoxide ; TFA: trifluoroacetic acid ; THF: tetrahydrofuran.

**2-(Cholesteryloxycarbonyl)aminoethanol (2):** A solution of cholesteryl chloroformate (99.89g, 0.218 mol) in CH₂Cl₂ (600 mL) was added to a stirred solution of 2-aminoethanol (29.5 mL, 0.489 mol, 2.2 equiv) in CH₂Cl₂ (450 mL) at 0°C over a period of 2 hours. The reaction was allowed to warm to room temperature and stirring continued for a further 14h. The reaction mixture was washed with saturated NaHCO₃ (2*200mL), water (2*200mL), dried (MgSO₄) and the solvents removed under reduced. The solid obtained was recrystallised (CH₂Cl₂/MeOH) to give 2 as a white solid. Yield: 99.67g (97%) ; m.p. : 180°C; R_{f}= 0.26 (acetone/ether 1:9); IR (CH₂Cl₂): *v*ₘₐₓ= 3353, 2942, 2870, 1693, 1674, 1562, 1467, 1382, 1264 cm⁻¹; ¹H NMR (270 MHz, CDCl₃): δ=5.35 (d, *J*=6.5 Hz, 1H, H6'), 5.25-5.29 (m, 1H, NH), 4.42-4.57 (1H, m, H3'), 3.70-3.62 (m, 2H, H1), 3.25-3.35 (m, 2H, H2), 3.12 (s, 1H, OH), 2.28-2.38 (m, 2H, H4'), 1.77-2.03 (m, 5H, H2', H7', H8'), 1.59-0.96 (m, 21H, H1', H9', H11', H12', H14'-H17', H22'-H25'), 1 (3H, s, H-19'), 0.9(d, *J=*6.5Hz, 3H, H21'), .87 (d, *J*=6.5 Hz, 6H, H26'&H27') and .67 (s, 3H, H18'); MS (FAB⁺): *m*/*z* = 496 [M+Na]⁺, 474 [M+H]⁺, 369[Chol]⁺, 255,175,145,105,95,81,43.

**2-[(Cholesteryloxycarbonyl)amino]ethyl methanesulfonate (3):**To a solution of 2 (25g, 52.3 mmol) and triethylamine (22 mL, 0.16 mol, 3 equiv) in CH₂Cl₂ (500 mL) at 0°C, was added dropwise a solution of methanesulfonyl chloride (10.5 mL, 0.13 mol, 2.5 equiv). The reaction mixture was allowed to warm at room temperature and stirred for 1h30. After Tlc analysis has indicated that the reaction had gone to completion, ice was added to quench the reaction. The reaction mixture was added to saturated aqueous NH₄Cl (600 mL), and extracted with ether (3*300 mL). The combined organic layers were washed with water (2*300mL), brine (250 mL) and dried (Na₂SO₄). The solvent was remove under reduced pressure to give a white solid, which on purification by chromatography (ether) gave 3. Yield: 28.3g (98 %); IR (CH₂Cl₂): νₘₐₓ = 3453, 3342, 1716, 1531, 1377, 1137 & 798 cm⁻¹; ¹H NMR (270 MHz, CDCl₃): δ= 5.34 (d, *J*=6.5 Hz, 1H, H6'), 5-5.1 (m, 1H, NH), 4.41-4.53 (1H, m, H3'), 4.29-4.25 (t, *J*=5 Hz, 2H, H1), 3.47-3.52 (m, 2H, H2), 3.01 (s, 3H, H3), 2.24-2.36 (m, 2H, H4'), 1.74-2 (m, 5H, H2', H7', H8'), 0.9-1.6 (m, 21H, H1', H9', H11', H12', H14'-H17', H22'-H25'), 0.98 (3H, s, H-19'), 0.84(d, *J* = 6.5 Hz, 3H, H21'), .83 (d, *J* = 6.5 Hz, 6H, H26'&H27') and .65 (s, 3H, H18'); MS (FAB⁺): m/z = 1104[2M+H]⁺, 574 [M+Na]⁺, 552 [M+H]⁺, 369[Chol]⁺, 255,175,145,95,81.

**4-aza-N**^{**6**}**(cholesteryloxycarbonylamino) hexanol (4):** To a stirred solution of 3 (28,3 g, 51 mmol) dissolved in a minimum amount of THF, was added amino-propanol (160 mL, 2 mol, 39 equiv). Once Tlc indicated reaction completion (12h), CHCl₃ (350 mL) and K₂CO₃ (20 g) were added and the solution was vigorously stirred for 30 min. The suspension was then filtered through a short pad of Celite®, washing thoroughly with CHCl₃. This was washed with a saturated solution of Sodium Hydrogenocarbonate and dried (Na₂CO₃). The solvent was removed to give 4 as a white solid. Yield: 26.1 g (96 %); IR (CH₂Cl₂): νₘₐₓ=3350-3210, 2937, 2850, 1531, 1460, 1380, 1220, 1120, 1040 cm⁻¹; ¹H NMR (270 MHz, CDCl₃): δ= 5.33-5.35 (m, 1H, H6'), 4.92-4.96 (m, 1H, NH), 4.42-4.51 (1H, m, H3'), 3.7-3.83. (m, 2H, H5), 3.23-3.29 (m, 2H, H1), 2.73-2.57 (m, 6H, H2, H3, H4), 2.2-2.36 (m, 2H, H4'), 1.7-2 (m, 5H, H2', H7', H8'), 0.85-1.58 (m, 21H, H1', H9', H11', H12', H14'-H17', H22'-H25'), 0.98 (3H, s, H-19'), 0.84 (d, *J* = 6.5 Hz, 3H, H21'), .8 (d, *J* = 6.5 Hz, 6H, H26'&H27') and 0.61 (s, 3H, H18'); MS (FAB⁺): *m*/*z* = 543 [M+Na]⁺, 530 [M+H]⁺, 485 [M-CO₂]⁺, 369[Chol]⁺, 144 [M-Ochol]⁺,69,55.

**4-aza-(Boc)-N**^{**6**}**(cholesteryloxycarbonyl amino) hexanol (5):** To a solution of 4 (26.1g, 49 mmol), was added Et₃N (8.3 mL, 1.1 equiv) and Boc₂O (10.7g, 1 equiv) in CH₂Cl₂ (200 mL) and the resulting solution followed by tlc. On completion, the reaction mixture was poured into NH₄Cl (100 mL), and was washed with water and dried (Na₂SO₄). The solvent was removed *in vacuo* to give the white solid 5. The solvent was remove under reduced pressure to give a white solid, which on purification by chromatography (CH₂Cl₂/MeOH/NH₃ 92:7:1) gave 3. Yield (27.9 g, 90%); IR (CH₂Cl₂): νₘₐₓ= 3352, 3054, 2937, 1675, 1530, 1455, 1380, 1220, 1120; ¹H NMR (270 MHz, CDCl₃): δ= 5.33-5.35 (m, 1H, H6'), 4.86 (m, 1H, NH), 4.42-4.5 (1H, m, H3'), 3.62-3.7 (m, 2H, H5), 3.27-3.38 (m, 6H, H1, H2, H3), 2.18-2.33 (m, 2H, H4'), 1.73-2 (m, 5H, H2', H7', H8'), 1.45 (s, 9H, Boc), 1-1.65 (m, 23H, H4, H1', H9', H11', H12', H14'-H17', H22'-H25'), 0.97 (3H, s, H-19'), 0.93 (d, *J* = 6.5 Hz, 3H, H21'), 0.8 (d, *J* = 6.5 Hz, 6H, H26'&H27') and 0.65 (s, 3H, H18'); MS (FAB⁺): *m*/*z* = 654 [M+Na]⁺, 543 [M-Boc]⁺, 369[Chol]⁺, 145, 121, 95, 69,57.

**4-aza-(Boc)-N**^{**6**}**(cholesteryloxycarbonylamino) hexyl methane-sulfonate (6):** This experiment was carried out in a similar way as the preparation of 2-[(Cholesteryloxycarbonyl)amino]ethyl methanesulfonate 3 on 44 mmol scale giving 6. Yield (28g, 90%); IR (CH₂Cl₂): νₘₐₓ= 3305, 2980, 2900, 2865, 1675, 1530, 1455, 1350, 1150; ¹H NMR (270 MHz, CDCl₃): δ= 5.33-5.35 (m, 1H, H6'), 4.86 (m, 1H, NH), 4.35-4.55 (m, 1H, H3'), 4.22 (t, 2H, J = 6.5 Hz, H5), 3.2-3.4 (m, 6H, H1, H2, H3), 3.01(s, 3H, H6), 2.15-2.33 (m, 2H, H4'), 1.73-2 (m, 5H, H2', H7', H8'), 1.44 (s, 9H, Boc), 1-1.67 (m, 23H, H4, H1', H9', H11', H12', H14'-H17', H22'-H25'), 0.97 (3H, s, H-19'), 0.94 (d, *J* = 6.5 Hz, 3H, H21'). 0.8 (d, *J* = 6.5 Hz, 6H, H26'&H27') and 0.65 (s, 3H, H18'); MS (FAB⁺): *m*/*z* = 722 [M+Na]⁺, 609 [M-Boc]⁺ 369[Chol]⁺, 145, 121, 95, 69,55.

**4-aza-(Boc)-N**⁶**(cholesteryloxycarbonylamino) hexanamine (7):** To 6 (25g, 35 mmol), sodium azide (11.49, 175.7 mmol, 5 equiv), and sodium iodine (5g, 35 mmol, 1 equiv) under nitrogen was added anhydrous DMF (200 mL), with stirring. Equipped with a reflux condenser, heating at 80°C for 2h resulted in completion of reaction. The reaction mixture was allowed to cool to room temperature, the DMF removed under reduced pressure and the residue dissolved in EtOAc. This was washed with water (2*100 mL), brine (100 mL) and dried (Na₂SO₄) to give after purification by chromatography (hexane/ether 1:1) 7 as a white solid. Yield (22g, 95 %); ¹H NMR (270 MHz, CDCl₃): δ= 5.34-5.36 (m, 1H, H6'), 4.35-4.55 (m, 1H, H3'), 4.25 (t, 2H, *J* = 6.5 Hz, H5), 3.2-3.5 (m, 6H, H1, H2, H3), 2.25-2.33 (m, 2H, H4'). 1.7-2.05 (m, 5H, H2', H7', H8'), 1.45 (s, 9H, Boc), 1-1.72 (m, 23H, H4, H1', H9', H11', H12', H14'-H17', H22'-H25'), 0.98 (3H, s, H-19'), 0.94 (d, *J* = 6.5 Hz, 3H, H21'), 0.83 (d, *J* = 6.5 Hz, 6H, H26'&H27') and 0.64 (s, 3H, H18'); MS (FAB⁺): *m*/*z* = 568 [M+Na-Boc]⁺, 556 [M-Boc]⁺, 369[Chol]⁺, 145, 121, 95, 69,57.

**4-aza-(Boc)-N**^{**6**}**(cholesteryloxycarbonylamino) hexylamine (8):** To a round bottomed flask charged with 7 (22.75 g, 34,6 mmol) in THF (230 mL) was added trimethylphosphine in THF (1 M, 40 mL, 1.15 equiv), and the reaction was monitored by tlc. On the completion the reaction was stirred with water (3 mL) and aqueous ammonia (3mL) for 1h and the solvent was remove under reduce pressure. After chromatography (CH₂Cl₂/MeOH/NH₃ 92:7:1 to 75:22:3) 8 was obtained as a white crystal. Yield (19.1 g, 88 %); IR (CH₂Cl₂): νₘₐₓ= 3689, 3456, 3155, 2948, 2907, 2869, 2253, 1793 , 1709, 1512, 1468, 1381, 1168; ¹H NMR (270 MHz, CDCl₃): δ= 5.32-5.35 (m, 1H, H6'), 4.35-4.51 (m, 1H, H3'), 3.45-3.05 (m, 8H, H1, H2, H3, H5), 2.18-2.4 (m, 2H, H4'), 1.8-2.1 (m, 5H, H₂', H7', H8'), 1.46 (s, 9H, Boc), 1.01-1.72 (m, 23H, H4, H1', H9', H11', H12', H14'-H17', H22'-H25'), 0.97 (3H, s, H-19'), 0.85 (d, *J* = 6.5 Hz, 3H, H21'), 0.82 (d, *J* = 6.5 Hz, 6H, H26'&H27') and 0.64 (s, 3H, H18'); MS (FAB⁺): *m*/*z* = 630 [M+H]⁺, 530 [M-Boc]⁺, 369[Chol]⁺, 145, 121, 95, 69,57.

(**Boc)aminooxyacetic acid (9):** O-(Carboxymethyl)hydroxylamine hemihydrochloride (1.16 g, 5.3 mmol) was dissolved in CH₂Cl₂ (40 mL) and the pH was adjusted to 9 by addition of triethylamine (3 mL). Then di-tert-butyl dicarbonate (2.36 g, 10.6 mmol, 2.0 equiv) was added and the mixture was stirred at room temperature until tlc indicated completion of reaction. The pH was lowered to 3 by addition of diluted HCl. The reaction mixture was partitioned between saturated aqueous NH₄Cl (20 mL) and CH₂Cl₂ (30 mL). The aqueous phase was extracted with CH₂Cl₂ (3x100 mL). The combined organic extracts were washed with H₂O (2x100 mL) and dried (Na₂SO₄). The solvent was removed *in vacuo* to afford 9 as a white solid. Yield (1.86 g, 97%); IR (CH₂Cl₂): νₘₐₓ= 3373, 2983, 2574, 2461, 1724, 1413, 1369, 1235; ¹H NMR (270 MHz, CDCl₃): δ= 4.48 (s, 2H, CH₂), 1.48 (s, 9H, Boc); MS (FAB⁺): *m*/*z* =214 [M+Na]⁺, 192 [M+H]⁺, 135, 123, 109, 69.

**(Boc)aminooxy compound (10):** N-hydroxysuccinimide (0.36 g, 3.13 mmol, 1 equiv), 9 (0.6 g, 3.13 mmol, 1 equiv), and N,N'-dicyclohexylcarbodiimide (0.68 g, 3.13 mmol, 1 equiv) were dissolved in EtOAc (90 mL), and the heterogeneous mixture was allowed to stir at room temperature overnight. The mixture was then filtered through a pad of Celite® to remove the dicyclohexylurea, which was formed as a white precipitate (rinsed with 60 mL of EtOAc), and added to a solution of 8 (1.97 g, 3.13 mmol, 1 equiv) in THF (10 mL). A pH of 8 was maintained for this heterogeneous reaction by addition of triethylamine (6 mL). The resulting mixture was allowed to stir at room temperature overnight. On completion the mixture was filtered and the solvent was removed under reduced pressure to give after purification by flash-chromatography (CH₂Cl₂/MeOH/NH₃ 92:7:1) 10 as a white solid. Yield (2.3 g, 90 %); ¹H NMR (270 MHz, CDCl₃): δ= 5.33-5.35 (m, 1H, H6'), 4.4-4.52 (m, 1H, H3'), 4.3 (s, 2H, H90, 3.2-3.42 (m, 8H, H1, H2, H4, H6), 2.23-2.35 (m, 2H, H4'), 1.7-2.1 (m, 7H, H2', H7'. H8', H5), 1.44-1.46 (m, 18H, 2 Boc), 1-1.73 (m, 21H, H1', H9', H11', H12', H14'-H17', H22'-H25'), 0.98 (3H, s, H-19'), 0.85 (d, *J* = 6.5 Hz, 3H, H21'), 0.83 (d, *J* = 6.5 Hz, 6H, H26'&H27') and 0.65 (s, 3H, H18'); MS (FAS): *m*/*z* =803 [M+H]⁺, 703 [M-Boc]⁺, 647, 603 [M-2Boc]⁺, 369, 279, 255, 235, 204, 145, 95, 69.

**Hydroxylamine (11):** To a solution 10 (1.1 g, 1.36 mmol, 1 equiv) in CH₂Cl₂ (10 mL) was added TFA (2 mL, 20.4 mmol, 15 equiv) at 0°C. The solution was allowed to stir at room temperature for 5 hours. On completion toluene was added to azeotrope TFA from the reaction mixture. The solvents were removed *in vacuo* to afford after purification by chromatography (CH₂Cl₂/MeOH/NH₃ 92:7:1 to 75:22:3) 11 as a white solid (709 mg, Yield: 86 %); IR (CHCl₃): νₘₐₓ= 3306, 2948, 2850, 2246, 1698, 1647, 1541, 1467, 1253, 1133; ¹H NMR (270 MHz, CDCl₃): δ=5.26-5.4 (m, 1 H, H6'), 4.4-4.52 (m, 1H, H3'), 4.12 (s, 2H, H9), 3.34-3.41 (m, 2H, H2), 3.15-3.3 (m, 2H, H4), 2.6-2.74 (m, 4H, H1 & H6), 2.14-2.39 (m, 2H, H4'), 1.62-2.1 (m, 7H, H2', H7', H8', H5), 1.02-1.6 (m, 21H, H1', H9', H11', H12', H14'-H17', H22'-H25'), 0.96 (3H, s, H-19'), 0.86 (d, *J*= 6.5 Hz, 3H, H21'), 0.83 (d, *J* = 6.5 Hz, 6H, H26'&H27') and 0.66 (s, 3H, H18'); MS (FAB⁺): *m*/*z* = 603 [M+H]⁺, 369[Chol]⁺, 160, 137, 109, 95, 81, 69, 55.

**Mannosyl compound (12a):** A solution of D-mannose (266 mg, 4.8 mmol) in Acetic aqueous Buffer (sodium acetate/acetic acid 0.1 M, pH 4, 7mL) and a solution of 11 (290 mg, 0.48 mmol, 10 equiv) in DMF (7 mL) was mixed and stirred for 3 days at room temperature. The solvent was removed *in vacuo* by freeze drying and chromatography (CH₂Cl₂/MeOH/NH₃ 75:22:3) afforded the product 21 a white solid (233 mg, Yield : 65 %). The purity was further confirmed by HPLC. The final product contained of the β-pyranose (82 %) form and α-pyranose (18 %) form that were not isolated but characterized in the mixture. MS (FAB⁺): m/z = 765 [M+H]⁺, 787 [M+Na]⁺, 397, 369[Chol]⁺, 322, 240, 121, 109, 95, 81, 69,57. *β-pyranose form.*¹H NMR (400 MHz, CD₃OD/CDCl3 [75/25]): δ= 7.64-7.62 (d, ³*J*₁ₐ₋₂ₐ = 7 Hz, 1H, H1a), 5.35-5.36 (m, 1H, H6'), 4.45-4.5 (s, 2H, H9), 4.35-4.5 (m, 1H, H3'), 4.19-4.24 (dd, 1H, H2ₐ, ³*J*₁ₐ₋₂ₐ = 7.4 Hz, ³*J*₂ₐ₋₃ₐ = 7.7 Hz), 3.81-3.9 (m, 1H, H3a), 3.73-3.8 (m, 2H, H4a, H6ₐₓa), 3.63-3.71 (m, 2H, H5a, H_{eq}6a), 3.34-3.42 (m, 2H, H2), 3.27-3.30 (m, 2H, H4), 3-3.08 (m, 2H, H1), 2.9-2.98 (m, 2H, H6), 2.25-2.35 (m, 2H, H4'), 1.78-2.07 (m, 7H, H2', H7', H8', H5), 1.03-1.65 (m, 21H, H1', H9', H11', H12', H14'-H17', H22'-H25'), 1.01 (3H, s, H-19'), 0.91 (d, *J* = 6.5 Hz, 3H, H21'), 0.85 (d, *J* = 6.5 Hz, 6H, H26'&H27') and 0.69 (s, 3H, H18'); ¹³C NMR (400MHz, CDCl₃/ CD₃OD [25/75]): 12.33 (C18'), 19.20 (C21'), 19.74 (C19'), 21.91 (C11'), 22.91 (C27'), 23.17 (C26'), 24.67 (C23'), 25.07 (C15'), 27.37 (C5), 28.85 (C25'), 28.96 (C2'), 29.07 (C12'), 32.76 (C7'), 32.87 (C8'), 36,38 (C2), 36.78 (C20'), 37.09 (C1) 37.76 (C22'),37.95 (C1'), 38.4 (C4), 39.36 (C4'), 40.41 (C24'), 40.76 (C16'), 46.16 (C6), 51.19 (C9'), 57.19 (C17'), 57.75 (C14'), 64.62 (C6a), 70.19 (C2a), 70.58 (C4a), 72.12 (C3a), 72.37 (C5a), 73.11 (C9), 75.91 (C3'), 123.39 (C6'), 140.72 (C5'), 155.02 (C1a), 158.69 (NHCOOChol), 173.1 (C8); *α-pyranose form:* identical data except, ¹H NMR (400 MHz, CD₃OD/CDCl3 [75/25]): δ= 6.90-6.88 (d, ³*J*₁ₐ₋₂ₐ = 7 Hz, 1H, H1a), 5-5.05 (dd, 1H, H2a, ³*J*₁ₐ₋₂ₐ = 7.3 Hz, ³*J*₂ₐ₋₃ₐ = 7.6 Hz); ¹³C NMR (400 MHz, CDCl₃/ CD₃OD [25/75]): 65.33 (C2a), 155.79 (C1a).¹H NMR (400 , CD₃OD/CDCl₃ [75/25]): (m, 1H, H3') missing, undemeath solvent peak; confirmed by ¹H NMR (300 MHz, DMSO): δ= 4.67-4.82 (m, 1H, H3'). ¹³C NMR (400 MHz, CDCl₃/ CD₃OD [25/75]): C1 missing, underneath MeOH peak confirmed by ¹H/¹³C correlation at 400 MHz, around 49. Proton resonance assignments were confirmed using ¹H gradient type DQF-COSY and TOCSY; ¹H/¹³C correlation and DEPT 135 were used to assign unambiguously the carbon resonances. a pyrannose form gave ¹*J*¹³C1a-H1a = 177 Hz and β pyrannose form gave ¹*J*¹³C1a-H1a = 167 Hz. ¹H phase-sensitive NOESY confirmed conformation.

**Glucosyl compound (12b):** This was prepared with a solution of D-glucose (150 mg, 0.82 mmol) and 11 (100 mg, 0.16 mmol) in a similar way to the preparation of 12a, stirred for 1 day and purified by chromatography (CH₂Cl₂/MeOH/NH₃ 75:22:3) to afford the product 12b as a white solid (103 mg, Yield: 82 %). The purity was further confirmed by HPLC. The final product contained of the α-pyranose (11 %) anomer and β-pyranose (89 %) anomer that were not isolated but characterized in the mixture. (FAB⁺): m/z = 765 [M+H]⁺, 787 [M+Na]⁺, 391, 369 [Chol]⁺, 309, 290, 171, 152, 135, 123, 109, 95, 81, 69 ; β-*pyranose form.* (300 MHz, CDCl₃/CD₃OD [90/10]): δ= 7.53-7.56 (d, *J* = 5.6 Hz, 1H, H1a), 5.26-5.36 (m, 1H, H6'), 4.2-4.45 (m, 3H, H9, H3'), 4.05-4.15 (m, 1H, H2a), 3.45-3.85 (m, 5H, H6a, H3a, H5a, H4a), 2.9-3.4 (m, H2, H4, MeOH), 2.9-3.15 (m, 4H, H1, H6), 2.15-2.3 (m, 2H, H4'), 1.65-2 (m, 5H, H2', H7', H8'), 0.95-1.55 (m, 23H, H5, H1', H9', H11', H12', H14'-H17', H22'-H25'), 0.93 (3H, s, H-19'), 0.84 (d, *J* = 6.5 Hz, 3H, H21'), 0.78 (d, *J* = 6.5 Hz, 6H, H26'&H27') and 0.62 (s, 3H, H18'); *α-pyranose form* : identical data except, ¹H NMR (300 MHz, CDCl₃/CD₃OD (90/10]): δ= 7.22-7.24 (d, *J* = 6,61 Hz, 1H, H1a), 4.95-5.07 (m, 1H, H2a); ¹H NMR (300 MHz, CD₃OD): (m, 1H, H3') missing, presumably underneath solvent peak; confirmed by ¹H NMR (300 MHz, DMSO): δ= 4.7-4.86 (m, 1H, H3')

**Galactosyl compound (12c):** This was prepared with a solution of D-galactose (50 mg, 0.27 mmol) and 11 (40 mg, 0.066 mmol in a similar way to the preparation of 12a, stirred for 1 day and purified by chromatography (CH₂Cl₂/MeOH/NH₃ 75:22:3) to afford the product 12c as a white solid (35 mg, Yield: 70 %). The purity was further confirmed by HPLC. The final product contained of the α-pyranose (15 %) form and *β*-pyranose (85 %) form that were not isolated but characterized in the mixture. MS (FAB⁺): *m*/*z* = 765 [M+H]⁺, 588, 391, 369 [Chol]⁺, 322, 290, 165, 152, 135, 121, 109, 95, 81, 69 ; *β-pyranose form*. ¹H NMR (270 MHz, DMSO): δ= 7.78-7.82 (m, 1H, NHCO of C8), 7.55-7.58 (d, *J* = 7.2 Hz, 1H, H1a), 6.95-7.1 (m, 1H, NHCOOChol), 5.25-5.37 (m, 1H, H6'), 4.2-4.43 (m, 3H, H9, H3'), 3.2-3.9 (m, H2a, H6a, H3a, H5a, H4a, OH), 2.9-3.18 (m, 4H, H2, H4), 2.4-2.65 (m, 4H, H1, H6), 2.15-2.3 (m, 2H, H4'), 1.67-2 (m, 5H, H2', H7', H8'), 0.92-1.6 (m, 23H, H5, H1', H9', H11', H12', H14'-H17', H22'-H25'), 0.96 (3H, s, H-19'), 0.89 (d, *J* = 6.5 Hz, 3H, H21'), 0.84 (d, *J* = 6.5 Hz, 6H, H26'&H27') and 0.65 (s, 3H, H18'). *α-pyranose form :* identical data except, ¹H NMR (270 MHz, DMSO): 6.86-6.88 (d, *J* = 6 Hz, 1H, H1a) Glucuronic compound (12d): This was prepared with a solution of D-glucuronic acid, sodium salt monohydrate (30 mg, 0.128 mmol, 1.5 equiv) and 11 (50 mg, 0.08 mmol) in a similar way to the preparation of 12a, stirred for 1 day, purified by chromatography (CH₂Cl₂/MeOH/NH₃ 75:22:3) to afford the sodium salt of 12d as a white solid (41 mg, Yield: 60 %). The purity was further confirmed by HPLC. The final product contained of the α-pyranose (85 %) form and β-pyranose (15 %) form that were not isolated but characterized in the mixture. MS (FAB⁺): *m*/*z* = 779 [M+H]⁺, 733, 588, 411, 369[Chol]⁺, 336, 290, 240, 214, 159, 145, 135, 121, 109, 95, 81, 69,55. *β-pyranose form.* ¹H NMR (300 MHz, CDCl₃/CD₃OD [75/25]) : δ= 7.51-7.53 (d, *J* = 5.9 Hz, 1H, H1a), 5.25-5.33 (m, 1H, H6'), 4.2-4.45 (m, 3H, H9, H3'), 3.8-4.1 (m, 3H, H2a, H3a, H4a), 3.6-3.75 (m, 1H, H5a), 3.2-3.55 (m, H2, H4, MeOH), 2.7-3.15 (m, 4H, H1, H6), 2.18-2.32 (m, 2H, H4'), 1.62-2 (m, 5H, H2', H7', H8'), 0.9-1.6 (m, 23H, H5, H1', H9', H11', H12', H14'-H17', H22'-H25'), 0.93 (3H, s, H-19'), 0.83 (d, *J* = 6.5 Hz, 3H, H21'), 0.77 (d, *J* = 6.5 Hz, 6H, H26'&H27') and 0.6 (s, 3H, H18')); *α-pyranose form:* identical data except, ¹H NMR (300 MHz, CD₃OD): δ= 7.22-7.24 (d, *J* = 6.3 Hz, 1H, H1a), 5-5.1 (m, 1H, H2a).

**β-D-lactosyl compound (12e):** A solution of β-D-Lactose, containing 25-30 % of a (1.13 g, 3.3 mmol) and 11 (200 mg, 0.33 mmol) in 14 mL of DMF/Acetic aqueous Buffer was stirred for 4 days at room temperature. The solvent was removed *in vacuo* by freeze-drying and chromatography (CH₂Cl₂/MeOH/NH₃ 75:22:3) afforded the product 12e as a white solid (145 mg, Yield: 47 %). The purity was further confirmed by HPLC. The final product contained of the α-pyranose (15 %) form and β-pyranose (85 %) form (containing itself around 25 % of α lactose) that were not isolated but characterized in the mixture. MS (FAB⁺): m/z = 927 [M+H]⁺, 588, 482, 369[Chol]⁺, 290, 243, 216, 178, 152, 135, 121, 109, 95, 81, 69,55 ; β-*pyranose* form. ¹H NMR (400 MHz, CDCl₃/ CD₃OD [20/80]): δ_{H}= 7.69-7.71 (d, ³*J*₁ₐ₋₂ₐ = 5.8 Hz, 1 H, H1a of β lactose), 7.66-7.68 (d, ³*J*₁ₐ₋₂ₐ= 6.2 Hz, 1H, H1a of a lactose), 5.35-5.37 (m, 1H, H6'), 4.37-4.6 (m, 4H, H9, H3', H2a), 4.2-4.37 (m, 1H, H1b), 3.65-4.05 (m, 7 H, H3a, H4a, H5a, H4b, H5b, H6b), 3.25-3.6 (m, 8H, H2, H4, H6a, H2b, H3b,MeOH), 3-3.2 (m, 4H, H1, H6), 2.25-2.42 (m, 2H, H4'), 1.8-2.15 (m, 5H, H2', H7', H8'), 1-1.65 (m, 23H, H5, H1', H9', H11', H12', H14'-H17', H22'-H25'), 1.01 (3H, s, H-19'), 0.91 (d, *J* = 6.5 Hz, 3H, H21'), 0.85 (d, *J* = 6.5 Hz, 6H, H26'&H27') and 0.69 (s, 3H, H18'); ¹³C NMR (400 MHz, CDCl₃/ CD₃OD [20/80]): ¹³C NMR (400 MHz, CDCl₃/ CD₃OD [20/80]): 12.32 (C18'), 19.2 (C21'), 19.76 (C19'), 21.94 (C11'), 22.91 (C27'), 23.17 (C26'), 24.7 (C23'), 25.1 (C15'), 27.22 (C5), 28.89 (C25'), 29 (C2'), 29.1 (C12'), 32.8 (C7'), 32.92 (C8'), 36.29 (C22'),36.81 (C10'), 37.12 (C1'). 37.99 (C6), 38.11 (C1), 39.48 (C2), 40.45 (C24'), 40.80 (C16'), 46.13 (C4'), 51.23 (C9'), 57.22 (C17'), 57.80 (C14'), 62.41 (C6a), 63.4 (C6a), 70.02 (C5b), 70.63 (C2a), 72.8 (C3a), 73 (C3'), 73.18 (C9), 74,75 (C2b), 76.8 (C3a), 81 (C4b), 92.39 (C1b), 105.2 (C3'), 123.42 (C6'), 140.72 (C5'), 154.8 (C1a), 156.2 (NHCOOChol), 173.17 (C8).*α-pyranose form:* identical data except, ¹H NMR (400 MHz, CD₃OD/CDCl3 [80/20]): δ_{H}= 7.04-7.05 (d, ³*J*₁ₐ₋₂ₐ = 5.6 Hz, 1H, H1a), 5.05-5.07 (m, 1H, H2a), 4.09-4.11 (m, 1H, H3a);¹H NMR (270 MHz, CD₃OD): (m, 1H, H3') missing, presumably underneath solvent peak; confirmed by ¹H NMR (300 MHz, DMSO): δ= 4.7-4.85 (m, 1H, H3'). Proton resonance assignments were confirmed using ¹H gradient type DQF-COSY and TOCSY; ¹H/¹³C correlation and DEPT 135 were used to assign unambiguously the carbon resonances. ¹H phase-sensitive NOESY confirmed conformation.

**Maltosyl compound (12f):** This was prepared with a solution of D Maltose monohydrate (30 mg, 1.8 mmol, 5 equiv) and **11** (100 mg, 0.16 mmol)) in a similar way to the preparation of **12e**, stirred for 1 day and purified by chromatography (CH₂Cl₂/MeOH/NH₃ 75:22:3) to afford **12f** as a white solid (100 mg, Yield : 65 %). The purity was further confirmed by HPLC. The final product contained of the α-pyranose (87 %) form and β-pyranose (13 %) form that were not isolated but characterized in the mixture. MS (FAB⁺): m/z = 927 [M+H]⁺, 765, 588, 559, 484, 369[Chol]⁺, 322, 290, 213, 167, 161, 143, 135, 121, 109, 95, 81, 69,55. *β-pyranose form.* ¹H NMR (300 MHz, CDCl₃/CD₃OD [80/20]): δ= 7.55-7.57 (d, ³J₁a-2a = 5.3 Hz, 1H, H1a), 5.3 (s, 1H, H6'), 4.85-5.02 (m, 1H, H3'), 4.09-4.22 (m, 1H, H1b), 3.57-4 (m, 7 H, H3a, H4a, H5a, H4b, H5b, H6b), 3.2-3.6 (m, 8H, H2, H4, H6a, H2b, H3b,MeOH), 2.8-3.1 (m, 4H, H1, H6), 2.1-2.36 (m, 2H, H4'), 1.6-2.05 (m, 5H, H2', H7', H8'), 1-1.6 (m, 23H, H5, H1', H9', H11', H12', H14'-H17', H22'-H25'), 0.93 (3H, s, H-19'), 0.83 (d, J = 6.5 Hz, 3H, H21'), 0.78 (d, *J* = 6.5 Hz, 6H, H26'&H27') and 0.6 (s, 3H, H18')*;α-pyranose form :* identical data except, ¹H NMR (300 MHz, CD₃OD/CDCl3 [80/20]): δ= 6.92-6.94 (d, *J* = 4.62 Hz, 1H, H1a), 5.02-5.15 (m, 1H, H2a), 4.04-4.08 (m, 1H, H3a)

**Maltotriosyl compound (12g):** This was prepared with a solution of maltotriose (246.4 mg, 0.46 mmol, 7 equiv) and **11** (40 mg, 0.066 mmol) in a similar way to the preparation of **12e**, stirred for 5 days and purified by chromatography (CH₂Cl₂/MeOH/NH₃ 75:22:3) to afford **12f** as a white solid (61 mg, Yield: 85 %). The purity was further confirmed by HPLC. The final product contained of the α-pyranose (15 %) form and β-pyranose (85 %) form that were not isolated but characterized in the mixture. MS (FAB⁺): *m*/*z* = 1111 [M+Na]⁺, 1089 [M+H]⁺, 588, 423, 391, 369 [Chol]⁺, 240, 171, 159, 145, 121, 105, 95, 81, 69; *β-pyranose form.:* ¹H NMR (300 MHz, CDCl₃/MeOH[20/80]): δ= 7.56-7.58 (d, *J* = 6 Hz, 1H, H1a), 5.2-5.27 (m, 1H, H6'), 4.9-4.95 (m, 1H, H3'), 4.2-4.45 (m, 4H, H9, H3', H2a), 4.05-4.2 (m, 2H, H1b, H1c), 2.95-4 (m, 21 H, H2, H4, H6a, H3a, H5a, H4a, H2b-6b, H2c-6c, MeOH), 2.85-2.95 (m, 4H, H1, H6), 2.2-2.3 (m, 2H, H4'), 1.8-2.1 (m, 5H, H2', H7', H8'), 0.98-1.6 (m, 23H, H5, H1', H9', H11', H12', H14'-H17', H22'-H25'), 0.94 (3H, s, H-19'), 0.84 (d, *J* = 6.5 Hz, 3H, H21'), 0.78 (d, *J* = 6.5 Hz, 6H, H26'&H27') and 0.61 (s, 3H, H18'); α-*pyranose* form: identical data except, ¹H NMR (300 MHz, CDCl₃/MeOH[20/80]): δ=6.85 (d, *J* = 5.6 Hz, 1H, H1a).

**Maltotetraosyl compound (12h):** This was prepared with a solution of D Maltotetraose (200 mg, 0.3030 mmol) and **11** (80 mg, 0.133 mmol, stirred for 5 days and purified by chromatography (CH₂Cl₂/MeOH/NH₃ 75:22:3) to afford **12h** as a white solid (67.5 mg, Yield : 41 %). The purity was further confirmed by HPLC. The final product contained of the α-pyranose (15 %) form and β-pyranose (85 %) form that were not isolated But characterized in the mixture. MS (FAB⁺): *m*/*z* = 1273 [M+Na]⁺, 1251 [M+H]⁺, 588, 369 [Chol]⁻, 159, 145, 121, 109, 95, 81, 69; HRMS (FAB⁺) C₅₅H₁₀₂N₄O₂₄Na: [M+Na]⁺ calcd 1273.6782, found 1273.6821. ***β**-pyranose form*.: ¹H NMR (300 MHz, CDCl₃/MeOH[20/80]): δ= 7.56-7.58 (d, 1H, H1a), 5.15-5.25 (m, 1H, H6'), 4.95-5.1 (m, 1H, H3'), 4.38-4.5 (m, 4H, H9, H3', H2a), 4.04-4.22 (m, 3H, H1b, H1c, H1d), 3.1-3.95 (m, 27H, H2, H4, H6a, H3a, H5a, H4a, H2b-6b, H2c-6c, H2d-6d, MeOH), 2.85-3.1 (m, 4H, H1, H6), 2-2-2.33 (m, 2H, H4'), 1.75-2.1 (m. 5H, H2', H7', H8'), 1-1.6 (m, 23H, H5, H1', H9', H11', H12', H14'-H17', H22'-H25'), 0.92 (3H, s, H-19'), 0.82 (d, *J* = 6.5 Hz, 3H, H21'), 0.78 (d, *J* = 6.5 Hz, 6H, H26'&H27') and 0.68 (s, 3H, H18'); *α-pyranose form:* identical data except. ¹H NMR (300 MHz, CDCl₃/MeOH[20/80]): δ= 7 (d, 1H, H1a).

**Maltohoptaosyl compound (12i):** This was prepared with a solution of D Maltoheptaose (100 mg, 0.08673 mmol) and 11 (30 mg, 0.0497 mmol) stirred for 7 days and purified by chromatography (CH₂Cl₂/MeOH/NH₃ 75:22:3) to afford 12i as a white solid (46mg, Yield: 53 %). The purity was further confirmed by HPLC. The final product contained of the α-pyranose (15 %) form and β-pyranose (85 %) form that were not isolated but characterized in the mixture. MS (FAB⁺): m/z = 1759 [M+Na]⁺, 1737 [M+H]⁻, 369 [Chol]⁺. 145, 121. 109, 95, 81. *β-pyranose form.:* ¹H NMR (300 MHz. CDCl₃/MeOH[20/80]): δ= 7.53-7.58 (d, 1H, H1a), 5.35-5.37 (m, 1H, H6'), 4.97-5.12 (m, 1H, H3'), 4.45-4.6 (m, 4H, H9, H3', H2a), 4-4.5 (m, 6H, H1b, H1c-g), 3.1-3.9 (m, 45H, H2, H4, H6a, H3a, H5a, H4a, H2b-6b, H2c-6c, H2d-6d. H2e-6e, H2f-6f, H2g-6g, MeOH), 2.7-3 (m, 4H, H1, H6), 2.15-2.35 (m, 2H, H4'), 1.7-2.1 (m, 5H, H2', H7', H8'). 1-1.6 (m, 23H, H5, H1', H9', H11', H12', H14'-H17', H22'-H25'), 0.94 (3H, s, H-19') 0.84 (d, *J* = 6.5 Hz, 3H, H21'), 0.77 (d, *J* = 6.5 Hz, 6H, H26'&H27') and 0.63 (s, 3H, H18'): *α-pyranose form:* identical data except. ¹H NMR (300 MHz, CDCl₃/MeOH[20/80]): δ=6.9 (d, 1H, H1a).

The following examples describe the preparation of compositions comprising a nucleotide sequence.

### Biological and biophysical evaluation:

**General:** Dioleoylphosphatidyl-ethanolamine (DOPE) was purchased from Avanti Lipid (Alabaster, Al, USA). Plasmid PCMVβ was produced by Bayou Biolabs (Harahan, LA, USA). DC-Chol was synthesised in our Laboratory. Mu-peptide was synthesised by M. Keller by standard Fmoc based *Merrifield* solid phase peptide chemistry on *Wang* resine. All other chemicals were reagent grade.

**Preparation of Liposomes:** DC-Chol (7.5 mg, 15 µmol) and DOPE (7.5 mg. 10 µmol) were combined in dichloromethane. The solution was transferred to a round-bottomed flask (typically 50 ml) and organic solvent removed under reduced pressure (rotary evaporator) giving a thin-lipid film that was dried for 2-3 h *in vacuo*. Following this, 4 mM HEPES buffer, pH 7.2 (3 ml) was added to the round-bottomed flask so as to hydrate the thin-lipid film. After brief sonication (2-3 min.) under argon, the resulting cationic liposome suspension (lipid concentration of 5 mg/ml) was extruded by means of an extruder device (Northern lipid). Initially, three times through two stacked polycarbonate filters (0.2 µm) and then ten times through two stacked polycarbonate filters (0.1 µm) to form small unilamellar cationic liposomes (average diameter 105 nm according to PCS analysis). Lipid concentrations (approx. 4-4.8 mg/ml) were determined by Stewart assay.

**Preparation of Liposome:Mu:DNA (LMD) and Liposome:DNA (LD) complexes:** Initially, mu:DNA (MD) particles were prepared by mixing as follows. Plasmid DNA stock solutions (typically 1.2 mg/ml) were added to a vortex-mixed, dilute solution of mu peptide (1 mg/ml) in 4mM HEPES buffer, pH 7.2. The final mu:DNA ratio was 0.6:1 w/w, unless otherwise stated, and final plasmid DNA concentration was 0.27 mg/ml. MD containing solutions were then added slowly under vortex conditions to suspensions of extruded cationic liposomes (typically approx. 4.5mg/ml), prepared as descnbed above, resulting in the formation of small LMD particles with narrow size distribution (120 ± 30 nm) as measured by PCS. Final lipid:mu:DNA ratio 12:0.6:1 w/w/w. A solution of sucrose (100 %, w/v) in 4mM HEPES buffer, pH 7.2, was then added to obtain LMD particle suspensions in 4mM HEPES buffer, pH 7.2 containing 10% w/v sucrose at the desired DNA concentration (final DNA concentration typically 0.14 mg/ml) and the whole stored at -80°C. Liposome:DNA (LD) complexes (lipoplexes) were prepared for experiments with a lipid:DNA ratio of 12:1 (w/w) folloWing the same protocol without the addition of Mu peptide.

**Particle size measurements:** The sizes of the lipoplexes were evaluated after 30 min exposure at 37° C to biological media by Photon Correlation Spectroscopy (N4 plus, Coulter). The chosen DNA particular concentration was compatible with in vitro condition (1 µg/ml of DNA). The parameters used were: 20° C, 0.089 cP, reflexive index of 1.33, angle of 90° C, 632.8 nm. Unimodal analysis was used to evaluate the mean particle size in Optimem. Size distribution program using the CONTIN algorithm was utilised to separate the sub-population of small serum particle of less than 50nm and to extracted the calculated size of lipoplexes in Optimem + 10% FCS.

**Transfection of HeLa cells:** Cells were seeded in a 24-wells culture plate In DMEM supplemented with 10% FCS and grown to approximately 70% confluence for 24h at 37°C in the presence of 5% CO₂. The cells were washed in PBS before the transfection media was administered to each well (0.5 ml of solution of 0.50 or 100 % Foetal Calf Serum in Dubelco OptiMem). 5 µl at 100 µg/ml DNA (nls βgal) of LMD were transfected onto Hella Cells for 30 min. Cells were then rinsed 3 times with PBS and incubated for a further 48h in DMEM supplemented with 10% FCS prior to processing for β-Gal expression by using standard chemiluminescent reporter gene assay kit (Roche Diagnostics, GmbH Cat No. 1758 241).

### Results and Discussion:

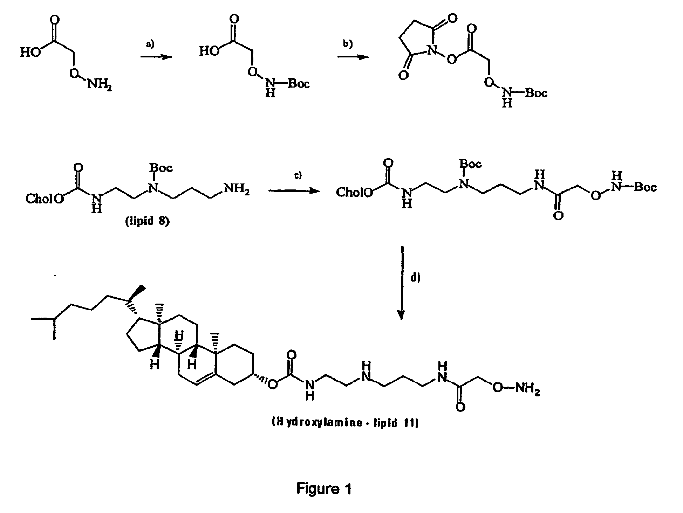

**Synthesis of Neoglycolipids:** Each member of the targeted family of neoglycolipids consisted of a cholesterol bearing lipid and an oligosaccharide molecule bound together via a linker. The whole synthetic approach was divided in two parts; firstly the synthesis of a lipid containing the linker and secondly the chemioselective coupling of this lipid with chosen sugar molecules. The key to this strategy is the formation of a hydroxylamine (Figure 1).

This synthesis of the Boc-protected lipid **(8)** was originally designed based on a convergent methodology using readily available aminoalcohols as starting materials with a complementary blocking group strategy for the amino group. This previously published methodology allowed the preparation of this polyamide-based lipid for gene transfer with little modification.

As mentioned, the glycosylation of hydroxylamino derivatives offers an elegant solution to our synthetic requirements. The commercially available O-(Carboxymethyl)hydroxylamine hydrochloride was first Boc-protected and then reacted with N-hydroxysuccinimide and N,N'-dicyclohexylcarbodilmide (DCC) resulting in the corresponding activated ester. This compound was treated immediately *in situ* with lipid **(8)** In THF at pH 8, affording a protected hydroxylamine. After a very straightforward deprotection with aqueous trifluoroacetic acid, the synthesis of the hydroxylamino lipid **(11)** was completed.

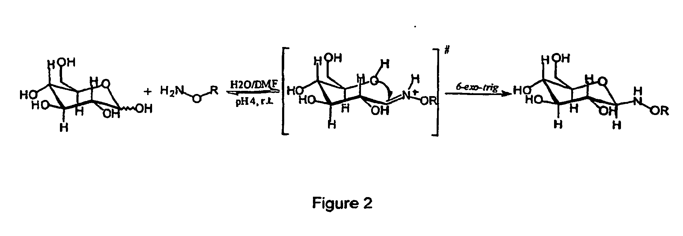

At this stage, we investigated the potential of our chemoselective coupling by reacting the lipid **(11)** with a number of commercially available non-protected oligosaccharides. This reaction was conducted under mild conditions using a solvent system of DMF and aqueous acetic acid pH 4 Buffer (1:1) which facilitates solubility of both sugar and lipid. As shown in Figure 2 the reactants are in dynamic equilibrium with the open chair protonated intermediate. In order to force the equilibrium to product formation, an excess of sugar was added. Due to the amphiphilic nature of the neoglycolipid product, isolation during workup was found to be difficult as a result of micelle and foam formation. Solubility problems also hampered the isolation, purification and analytical process. Reaction times and yields varied depending on the carbohydrate used (Table 1).

**Table 1 Yields, reaction times and diastereoselectivity of glycosylation of product 11.**

| Product | Sugar | Times (days) | Yield (%) | β/α |
|---|---|---|---|---|
| 12a | Mannose | 3 | 65 | 82/18 |
| 12b | Glucose | 1 | 80 | 89/11 |
| 12c | Galactose | 1 | 70 | 85/15 |
| 12d | Glucuronic acid | 1 | 60 | 85/15 |
| 12e | Lactose | 4 | 50 | 85/15 |
| 12f | Maltose | 1 | 65 | 87/13 |
| 12g | Maltotriose | 5 | 85 | 85/15 |
| 12h | Maltotetraose | 5 | 40 | 85/15 |
| 12i | Maltoheptaose | 7 | 55 | 85/15 |

**Neoglycolid Conformation:** Carbohydrate conformations can be ascertained by NMR in solution. The most useful data for conformation at the anomeric centre (C1a) is probably ¹J¹⁸C1a-H1a. The absolute value of this coupling constant depends upon the orientation of the carbon-hydrogen bond relative to the lone pairs of the ring oxygen, the electronegativity of the substituent at C1 and the nature of electronegative substituents attached to the rest of the molecule. The difference of ¹J¹⁸C1-H1 between α and β anomer of pyranoses can be used to determine the anomeric configuration. It is firmly established that ¹J(C1-H1eq) > ¹J(C1-H1ax) with an approximate difference of 10 Hz. ¹J(C1-H1eq) is usually around 170 Hz and ¹J(C1-H1ax) approximately 160 Hz. Higher values are observed when O-1 is exchanged with more electronegative element as chlorine or fluorine but a 10 Hz difference is usually observed. Carbon-hydrogen coupling constants of furanosides have been investigated and ¹J(C1-H1eq) > ¹J(C1-H1ax) but the difference is much smaller (1-3 Hz).

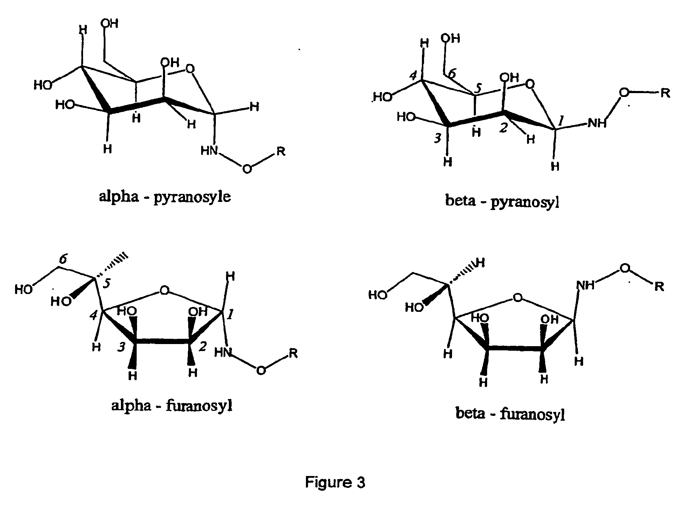

The characterization will be discussed based on the mannose example but the same analysis procedure was used for the other saccharides when NMR analysis conditions were favourable. Four distinct ring structures can be envisaged (Figure 3). The pyranose forms can be reasonably expected to be favoured over the furanose rings for steric reasons. So out of the two observed compounds in NMR, the main one is probably a pyranose. The secondary observed compound could not be attributed to mutarotation equilibrium because phase sensitive NOESY did not show a cross peak between the two C1a signals (proving it is a distinctive molecule). Therefore, this compound was not attributed to a furanose form because no shift of ¹⁹C5a was observed and ¹³C1a was not deshielded as has been demonstrated for related substituted furanose equivalents.

We measured ¹J¹³C1a-H1a = 167 Hz for the main compound and ¹J¹³ C1a-H1a = 177 Hz for the secondary one. The absolute value of those ¹J¹³C1-H1 is 10 Hz higher than expected for classical ⁴C₁ conformation but this is explained by the extreme electronegativity of the O-substituted hydroxylamine group that could slightly deform the chair structure. For pyranose rings it has been established that [¹J(C1-H1eq) - ¹J(C1-H1ax)] = 10 Hz, therefore it can be easily concluded that the main compound is the β anomer (H1ax) and the secondary compound is the α anomer (H1eq).

¹H phase sensitive NOESY confirmed this conclusion. Nuclear Overhauser effect was observed between H1a and H2a & H3a for the main compound. Considering the above detailed structure, this compound could not be the α pyranosyl anomer because the equatorial H1 cannot interact in space with H3, whereas the β anomer is perfectly able to generate such interactions. No nuclear overhauser effect was observed for H1a of the secondary compound but this could be due to a lack of sensitivity. Hence, in accordance with data from ¹J¹²C1-H1 and NOESY analyses, we concluded that two mannose pyranose α/β forms (20/80) were produced.

The very similar anomeric (β/α) isomers ratio obtained for the neoglycolipids is not surprising (Table 1), all the sugars having an equatorial hydroxyl in C2 but mannose.

The ratio obtained for this last compound is surprising because the β anomer is usually reported as sterically less favourable than the a one. A possible explanation is that this reaction could be driven by some secondary interactions (Hydrogen bonding) between the sugar and the hydroxylamine linker, stabilizing the β anomer (this is consistent with the observation that the NMR signal of the β anomer is always much more deshielded than the a one). This anomeric mixture of synthesized glycolipids are not expected to affect greatly the researched biological properties of the liposomal constructs, therefore we did not attempt the tedious separation of those diasteroisomers by preparative high pressure liquid chromatography.

**Biological application:**The glyco-modification of LMD was based on the natural ability of miscellar suspension to incorporate into lipid membranes. Firstly LMD were formulated following standard protocol and secondly a suspension of synthesized neoglycolipids miscelles in Hepes Buffer 4mM pH 7 was added to the LMD and incubated for 30min at room temperature before usual -80°C storage. Different percents of all the neoglycolipids produced were tested for stabilization effect but only the longer chain (maltotetraose **12h** and maltoheptaose **12i**) exhibited significant properties at less than 10 % (data not shown).

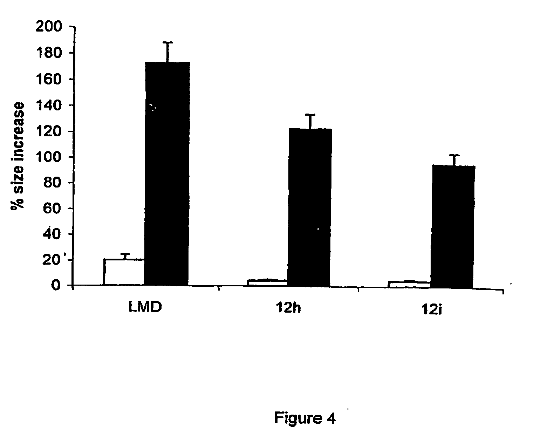

The stabilisation effect of neoglycolipid modified LMD was demonstrated by incorporation of 7.5 molar % of compound **12h** or **12i**. Lipid layers of liposomes based formulation are known to aggregate after salt or serum exposure. This phenomenon can be followed by measuring the average particle size increase after a fixed time; any stabilization of the LMD particle should be reflected in a reduction of this parameter. It was chosen to measure the size of the lipoplexes by Photon Correlation Spectroscopy (PCS) after 30 min exposure at 37°C to OptiMem or OptiMem + 10% FCS to mimic standard *in vitro* conditions. It was not possible to analyse the effect with PCS at higher serum percentages, the conditions being too extreme to allow for the taking of meaningful measurements. Figure 4 describes the percentage of size increase of those lipoplexes.

The results indicate a dear stabilisation of the partide between LMD and standard liposome formulation. Neoglycolipids introduction at 7.5% proved significantly beneficial in OptiMem and 10% serum. **12i** incorporation proved to be the most efficient. This result indicates the need of long carbohydrate chains to create efficient molecular brushes on top of those cationic lipid layers.

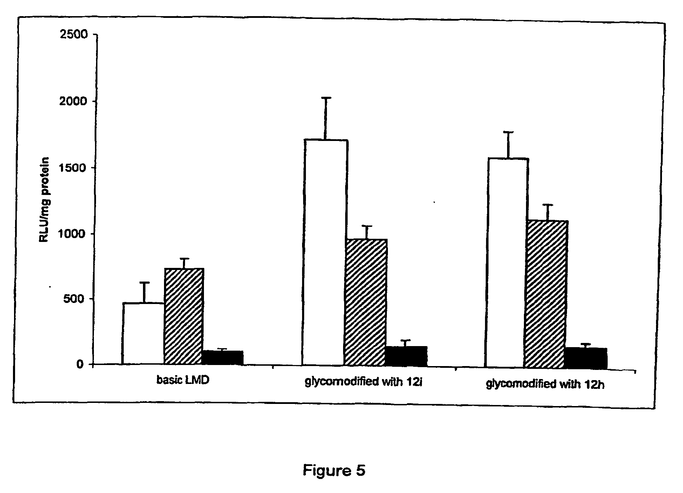

Even if some degree of stabilization is demonstrated, usually it results in a reduction of the affinity of the positively charged LMD for the negatively charged cell membrane, inducing a drop in the transfection ability of the construct. However in this case, the *in-vitro* transfection results indicated an enhancement of the transfection efficiency due to neoglycolipid modification in both 0% and 50% Serum condition (Figure 5). This result was attributed to a short range protective effect due to these neoglycolipids hindering short range van der waals based interactions between lipid bilayers of similar polarities but not affecting the longer range charge interactions between oppositely charged membranes. The aggregation induced by serum being based primarily on interaction of LMD with negatively charged proteins, the beneficial effect of our neoglycolipids was also lowered with an increasing percentage of serum (no significant benefit in 100% serum).

## Claims

1. A process for preparing a compound of the formula 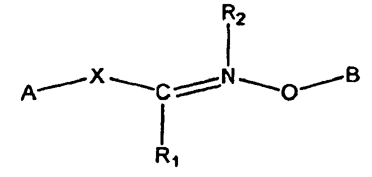 comprising reacting
(i) a compound of the formula; and 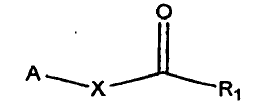
(ii) a compound of the formula 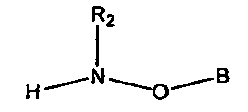
in admixture with or associated with a nucleotide sequence, or a pharmaceutically active agent;
wherein B is a lipid and A is a moiety of interest (MOI);
wherein X is an optional linker group;
wherein R₁ is H or a hydrocarbyl group; and
wherein R₂ is a lone pair or R₄, wherein R₄ is a suitable substituent.

2. A process according to claim 1 wherein the reaction is performed in an aqueous medium.

3. A composition comprising
(i) a compound of the formula 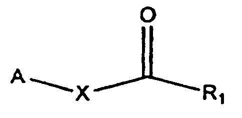
(ii) a compound of the formula 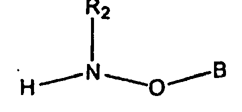 and
(iii) a nucleotide sequence, or a pharmaceutically active agent;
wherein B is a lipid and A is a moiety of interest (MOI);
wherein X is an optional linker group;
wherein R₁ is H or a hydrocarbyl group; and
wherein R₂ is a lone pair or a suitable substituent.

4. The invention according to any one of claims 1 to 3 wherein X is present.

5. The invention according to any one of claims 1 to 3 wherein X is a hydrocarbyl group.

6. The invention of any one of claims 1 to 5 wherein R₂ is a hydrocarbyl group

7. The invention of claim 6 wherein R₂ is a hydrocarbyl group containing optional heteroatoms selected from O, N and halogens.

8. The invention of any one of claims 1 to 5 wherein R₂ is H.

9. The invention according to any one of claims 1 to 8 wherein R₂ is H

10. The invention according to any one of claims 1 to 9 wherein the C=N bond is acid labile or acid resistant.

11. The invention according to claim 10 wherein the C=N bond is acid labile.

12. The invention according to claim 10 wherein the C=N bond is acid resistant.

13. The invention according to any one of the preceding claims wherein the lipid is or comprises a cholesterol group

14. The invention according to claim 13 wherein the cholesterol group is cholesterol.

15. The invention according to claim 13 wherein the cholesterol group is linked to X *via* a carbamoyl linkage or an ether linkage.

16. The invention according to any one of claims 1 to 15 wherein the lipid linked to X via a polyamine group.

17. The invention according to claim 16 wherein the polyamine group is not a naturally occurring polyamine.

18. The invention according to claim 16 or 17 wherein the polyamine group contains at least two amines of the polyamine group a re s paced from each other by an ethylene (-CH₂CH₂-) group.

19. The invention according to claim 16 wherein the polyamine is any one of spermidine, spermine or caldopentamine.

## Patentansprüche

1. Verfahren zur Herstellung einer Verbindung mit der Formel 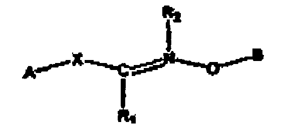 welches die Umsetzung
(i) einer Verbindung der Formel 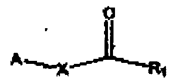 und
(ii) einer Verbindung der Formel 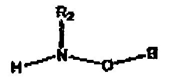
unter Beimengung von oder in Assoziierung mit einer Nukleotidsequenz oder eines pharmazeutisch aktiven Stoffes umfaßt,
worin B ein Lipid und A ein interessierender Rest (MOI) ist,
worin X eine optionale Linker-Gruppe ist,
worin R₁ H oder eine Kohlenwasserstoffgruppe ist und
worin R₂ ein einzelnes Elektronenpaar oder R₄ ist, wobei R₄ ein geeigneter Substituent ist.

2. Verfahren gemäß Anspruch 1, wobei die Reaktion in einem wässrigen Medium durchgeführt wird.

3. Zusammensetzung, die
(i) eine Verbindung der Formel 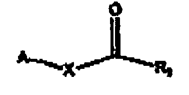
(ii) eine Verbindung der Formel 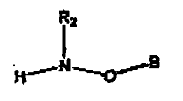 und
(iii) eine Nukleotidsequenz oder einen pharmazeutisch aktiven Stoff umfaßt,
worin B ein Lipid ist und A ein interessierender Rest (MOI) ist,
worin X eine optionale Linker-Gruppe ist,
worin R₁ H oder eine Kohlenwasserstoffgruppe ist und
worin R₂ ein einzelnes Elektronenpaar oder ein geeigneter Substituent ist.

4. Erfindung gemäß einem der Ansprüche 1 bis 3, worin X vorhanden ist.

5. Erfindung gemäß einem der Ansprüche 1 bis 3, worin X eine Kohlenwasserstoffgruppe ist

6. Erfindung von einem der Ansprüche 1 bis 5, worin R₂ eine Kohlenwasserstoffgruppe ist.

7. Erfindung von Anspruch 6, worin R₂ eine Kohlenwasserstoffgruppe ist, die optionale Heteroatome enthält, die unter O, N und Halogenen ausgewählt sind.

8. Erfindung von einem der Ansprüche 1 bis 5, worin R₂ H ist.

9. Erfindung gemäß einem der Ansprüche 1 bis 8, worin R₁ H ist

10. Erfindung gemäß einem der Ansprüche 1 bis 9, worin die C=N - Bindung säurelabil oder säurebeständig ist.

11. Erfindung gemäß Anspruch 10, worin die C=N - Bindung säurelabil ist

12. Erfindung gemäß Anspruch 10, worin die C=N - Bindung säurebeständig ist,

13. Erfindung gemäß einem der vorangegangenen Ansprüche, worin das Lipid eine Cholesteringruppe ist oder umfaßt.

14. Erfindung gemäß Anspruch 13, worin die Cholesteringruppe Cholesterin ist

15. Erfindung gemäß Anspruch 13, worin die Cholesteringruppe mit X über eine Carbamoyl-Verknüpfung oder eine Ether-Verknüpfung verbunden ist

16. Erfindung gemäß einem der Ansprüche 1 bis 15, worin das Lipid mit X über eine Polyamingruppe verbunden ist.

17. Verbindung gemäß Anspruch 16, worin die Polyamingruppe kein natürlich vorkommendes Polyamin ist,

18. Verbindung gemäß Anspruch 16 oder 17, worin die Polyamingruppe wenigstens zwei Amine der Polyamingruppe enthält, die voneinander durch eine Ethylengruppe (-CH₂CH₂ -) beabstandet sind.

19. Erfindung gemäß Anspruch 16, worin das Polyamin entweder Spermidin, Spermin oder Caldopentamin ist

## Revendications

1. Procédé pour la préparation d'un composé de formule 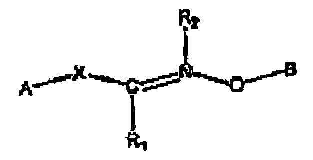 comprenant la réaction
(i) d'un composé de formule 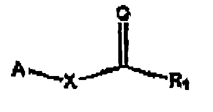 et
(ii) d'un composé de formule 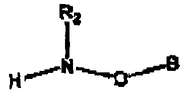
en mélange ou associé avec une séquence de nucléotides, ou un agent pharmaceutiquement actif ;
où B représente un lipide et A représente un groupement intéressant ("moiety of interest" MOI) ;
où X représente un groupe de liaison facultatif ;
où R₁ représente H ou un groupe hydrocarbyle ; et
où R₂ représente un doublet électronique libre ou un groupe R₄, R₄ représentant un substituant convenable.

2. Procédé suivant la revendication 1, dans lequel la réaction est conduite dans un milieu aqueux.

3. Composition comprenant
(i) un composé de formule 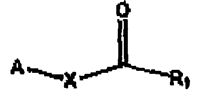
(ii) un composé de formule 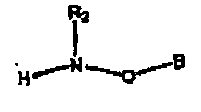 et
(iii) une séquence de nucléotides ou un agent pharmaceutiquement actif ;
où B représente un lipide et A représente un groupement intéressant (MOI) ;
où X représente un groupe de liaison facultatif ;
où R₁ représente H ou un groupe hydrocarbyle ; et
où R₂ représente un doublet électronique libre ou un substituant convenable.

4. Invention suivant l'une quelconque des revendications 1 à 3, dans laquelle X est présent.

5. Invention suivant l' une quelconque des revendications 1 à 3, dans laquelle X représente un groupe hydrocarbyle.

6. Invention suivant l'une quelconque des revendications 1 à 5, dans laquelle R₂ représente un groupe hydrocarbyle.

7. Invention suivant la revendication 6, dans laquelle R₂ représente un groupe hydrocarbyle contenant des hétéroatomes facultatifs choisis entre O, N et des halogènes.

8. Invention suivant l'une quelconque des revendications 1 à 5, dans laquelle R₂ représente H.

9. Invention suivant l'une quelconque des revendications 1 à 8, dans laquelle R₁ représente H.

10. Invention suivant l'une quelconque des revendications 1 à 9, dans laquelle la liaison C=N est labile en milieu acide ou résistante aux acides.

11. Invention suivant la revendication 10, dans laquelle la liaison C=N est labile en milieu acide.

12. Invention suivant la revendication 10, dans laquelle la liaison C=N est résistante aux acides.

13. Invention suivant l'une quelconque des revendications précédente, dans laquelle le lipide est ou comprend un groupe cholestérol.

14. Invention suivant la revendication 13, dans laquelle le groupe cholestérol est le cholestérol.

15. Invention suivant la revendication 13, dans laquelle le groupe cholestérol est lié à X par une liaison carbamoyle ou une liaison éther.

16. Invention suivant l'une quelconque des revendications 1 à 15, dans laquelle le lipide est lié à X par un groupe polyamine.

17. Invention suivant la revendication 16, dans laquelle le groupe polyamine n'est pas une polyamine naturelle.

18. Invention suivant la revendication 16 ou 17, dans laquelle le groupe polyamine contient au moins deux amines du groupe polyamine qui sont espacées l'une de l'autre par un groupe éthylène (-CH₂CH₂-).

19. Invention suivant la revendication 16, dans laquelle la polyamine est n'importe laquelle des polyamines consistant en spermidine, spermine et caldopentamine.
